(19) Europäisches Patentamt
European Patent Office
Office européen des brevets

(11) Numéro de publication: **0 012 639**
**B1**

# (12) FASCICULE DE BREVET EUROPEEN

(45) Date de publication du fascicule du brevet: **07.09.83**

(21) Numéro de dépôt: **79400871.4**

(22) Date de dépôt: **14.11.79**

(51) Int. Cl.³: **C 07 D 215/56**, C 07 D 417/12, C 07 D 401/12, C 07 D 413/12, A 61 K 31/47

(54) **Nouveaux dérivés de l'acide 3-quinoléine carboxylique, leur procédé de préparation, leur application comme médicament et les compositions pharmaceutiques les renfermant.**

(30) Priorité: **08.12.78 FR 7834592**

(43) Date de publication de la demande: **25.06.80 Bulletin 80/13**

(45) Mention de la délivrance du brevet: **07.09.83 Bulletin 83/36**

(84) Etats contractants désignés: **BE CH DE FR GB IT NL SE**

(56) Documents cités:
**FR-A-2 324 304**
**FR-A-2 340 735**
**JOURNAL OF HETEROCYCLIC CHEMISTRY vol. 2, no. 2, June 1965 Pennsylvania US A.S. DEY et al.: »Synthesis and properties of fluorine-containing heterocyclic compounds. I. Tri-fluoromethyl quinolines (1)«, pages 113–119**
**JOURNAL OF MEDICINAL CHEMISTRY vol. 22, no. 7, July 1979 P.P. PORTOGHESE Minnesota Univ. US ERICKSON et al.: »Inhibition of Rat Passive Cutaneous Anaphylaxis by 3-(Tetrazol-5-yl) quinolines« pages 816–823**

(73) Titulaire: **ROUSSEL-UCLAF, 102, route de Noisy Boîte postale no.9, F-93230 Romainville (FR)**

(72) Inventeur: **Clemence, François, 2, rue Turgot, F-75009-Paris (FR)**
Inventeur: **Deraedt, Roger, 23, Allée Jean-Baptiste Clément, F-93320 Pavillons-sous-Bois (FR)**
Inventeur: **Allais, André, 1, Allée Eugénie, F-93220 Gagny (FR)**
Inventeur: **Le Martret, Odile, 42, Avenue de Versailles, F-75016 Paris (FR)**

(74) Mandataire: **Vieillefosse, Jean-Claude et al, boîte postale no 9 102, route de Noisy, F-93230 Romainville (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



BUNDESDRUCKEREI BERLIN

# Nouveaux dérivés de l'acide 3-quinoléine carboxylique, leur procédé de préparation, leur application comme médicament et les compositions pharmaceutiques les renfermant

La présente invention concerne de nouveaux derivés de l'acide 3-quinoléine carboxylique, leur procédé de préparation, leur application comme médicament et les compositions pharmaceutiques les renfermant. Des dérivés de l'acide 3-quinoléine carboxylique, notamment des 4-hydroxy 8-trifluorométhyl 3-quinoléine carboxamide éventuellement substitués en 2 par un radical hydroxyle ou alcoyle ont été décrit dans le brevet FR-A-2 340 735. D'autres dérivés de l'acide 3-quinoléine carboxylique non substitués en 2 ont été décrits dans le brevet FR-A-2 324 304.

Les composés de la présente invention sont doués d'une activité anti-inflammatoire non négligeable, cette activité n'existant pas chez les composés des deux brevets mentionnés ci-dessus. Par ailleurs, certains des composés de la présente demande présentent une activité analgésique nettement supérieure à celle des composés précédents.

L'invention a pour objet les composés de formule I'

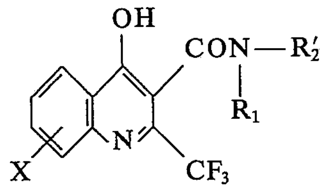

dans laquelle X' en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy, $R_1$ représente un atome d'hydrogène ou un radical alcoyle renfermant de 1 à 4 atomes de carbone, $R'_2$ représente un radical choisi parmi les radicaux thiazolyle, 4,5-dihydrothiazolyle, pyridinyle, oxazolyle, isoxazolyle, imidazolyle, pyrimidyle ou tétrazolyle, eventuellement substitués par un radical alcoyle renfermant de 1 à 4 atomes de carbone et phényle, éventuellement substitué, par au moins un radical choisi dans le groupe formé par les radicaux alcoyles renfermant de 1 à 4 atomes de carbone, les radicaux alcoxy renfermant de 1 à 4 atomes de carbone, le radical trifluorométhyle, le radical nitro et les atomes d'halogène, ainsi que leurs sels d'addition avec les acides.

L'invention a notamment pour objet les composés de formule I:

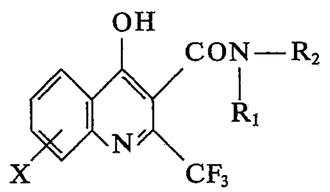

(I)

dans laquelle X en position 5, 6, 7 ou 8 représente un atome d'halogène, un radical trifluorométhyle, un radical trifluorométhylthio ou un radical trifluorométhoxy, $R_1$ représente un atome d'hydrogène ou un radical alcoyle renfermant de 1 à 4 atomes de carbone, $R_2$ représente un radical hétérocyclique choisi parmi les radicaux thiazolyle, 4,5-dihydrothiazolyle, pyridinyle, oxazolyle, isoxazolyle, imidazolyle ou pyrimidyle, éventuellement substitués par un radical alcoyle renfermant de 1 à 4 atomes de carbone ainsi que leurs sels d'addition avec les acides.

Lorsque $R_1$ représente un radical alcoyle, il s'agit de préférence du radical méthyle ou éthyle.

Lorsque X ou X' représente un atome d'halogène, il s'agit de préférence d'un atome de chlore.

Lorsque X' représente un radical alcoyle, il s'agit de préférence du radical méthyle, éthyle, n-propyle, n-butyle, n-pentyle, isopropyle ou isobutyle.

Lorsque X' représente un radical alcoxy, il s'agit de préférence du radical méthoxy, éthoxy ou n-propoxy.

Lorsque $R_2$ ou $R'_2$ représente un radical substitué par un radical alcoyle, il s'agit de préférence d'un radical substitué par un radical méthyle ou éthyle.

Lorsque $R'_2$ représente un radical phényle substitué, il s'agit de préférence d'un radical phényle substitué par au moins un radical choisi dans le groupe formé par les radicaux méthyle et éthyle, les radicaux méthoxy et éthoxy, le radical trifluorométhyle, le radical nitro et l'atome de chlore.

Parmi les sels d'addition avec les acides, on peut citer ceux formés avec les acides minéraux, tels que les acides chlorhydrique, bromhydrique, sulfurique ou phosphorique ainsi que ceux formés avec les acides sulfoniques, tels que les acides alcoyl ou arylsulfoniques comme, par exemple, l'acide méthanesulfonique ou paratoluènesulfonique.

L'invention a notamment pour objet les composés de formule I' pour lesquels X' représente un radical trifluorométhyle ainsi que leurs sels d'addition avec les acides, ceux pour lesquels X' est en

position 8, ainsi que leurs sels d'addition avec les acides, ceux pour lesquels $R_1$ représente un atome d'hydrogène ainsi que leurs sels d'addition avec les acides, ceux pour lesquels $R'_2$ représente un radical thiazolyle, ainsi que leurs sels d'addition avec les acides, ceux pour lesquels $R'_2$ représente un radical oxyzolyle ainsi que leurs sels d'addition avec les acides, ceux pour lesquels $R'_2$ représente un radical isocazolyle éventuellement substitué par un radical alcoyle renfermant de 1 à 4 atomes de carbone, et notamment le radical 5-méthyle isoxazolyle, ainsi que leurs sels d'addition avec les acides, ceux pour lesquels $R'_2$ représente un radical pyrimidyle ainsi que leurs sels d'addition avec les acides, ceux pour lesquels $R'_2$ représente un radical imidazolyle, éventuellement substitué par un radical alcoyle renfermant de 1 à 4 atomes de carbone et notamment le radical 1-méthyl 1H-imidazolyle, ainsi que leurs sels d'addition avec les acides, ainsi que les composés de formule I' pour lesquels $R'_2$ représente un radical tétrazolyle éventuellement substitué par un radical alcoyle renfermant de 1 à 4 atomes de carbone.

L'invention a naturellement tout spécialement pour objet les composés dont la préparation est donnée plus loin dans la partie expérimentale.

Les composés de formule I' ainsi que leurs sels, présentent d'intéressantes propriétés pharmacologiques et notamment une activité analgésique remarquable ainsi qu'une activité anti-inflammatoire, qui justifient leur utilisation en thérapeutique.

L'invention a donc pour objet les composés de formule I', tels que définis ci-dessus, ainsi que leurs sels avec les acides thérapeutiquement compatibles, à titre de médicaments.

L'invention a plus particulièrement pour objet à titre de médicaments le composé de l'exemple 1, ainsi que ses sels d'addition avec les acides thérapeutiquement acceptables.

Les médicaments, objets de l'invention peuvent être utilisés dans le traitement des algies musculaires, articulaires ou nerveuses, des douleurs dentaires, des migraines ainsi que dans le traitement des affections rhumatismales.

L'invention s'étend aux compositions pharmaceutiques renfermant comme principe actif, les médicaments définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie varie notamment en fonction de la voie d'administration, de l'affection traitée et du sujet en cause.

Par exemple, chez l'adulte, elle peut varier entre 20 mg et 2 g de principe actif par jour, par voie orale.

L'invention a également pour objet un procédé de préparation des composés de formule I' décrits ci-dessus, caractérisé en ce que l'on soumet un acide de formule II ou un dérivé fonctionnel de ce dernier

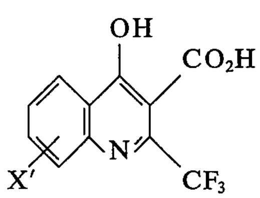

(II)

dans laquelle X' conserve la même signification que précédemment, à l'action d'un dérivé de formule III

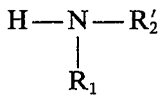

(III)

dans laquelle $R_1$ et $R'_2$ sont définis comme précédemment, pour obtenir le composé de formule I' correspondant que l'on soumet si désiré à l'action d'un acide pour en former le sel.

Dans un mode de réalisation préférée du procédé de l'invention:

- on utilise comme dérivé fonctionnel d'acide, un chlorure d'acide, un ester d'alcoyle inférieur, un anhydride ou un anhydride mixte;

– la condensation de l'acide de formule II ou du dérivé fonctionnel d'acide avec le composé de formule III a lieu au sein de la pyridine ou au sein d'un solvant inerte comme par exemple le benzène, le toluène ou l'acétate d'éthyle en présence d'un agent basique et de préférence en présence de triéthylamine.

Pour préparer le composé II, on soumet un composé de formule IV

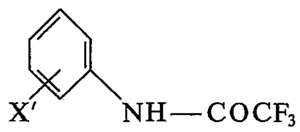

(IV)

dans laquelle X' conserve la même signification que précédemment, à l'action d'un agent de chloruration, obtient ainsi un composé de formule V:

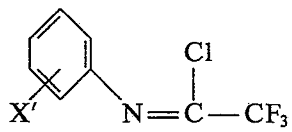

(V)

que l'on fait réagir avec un dérivé de formule

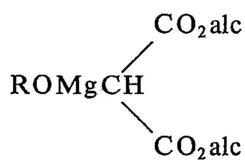

dans laquelle R et alc identiques ou différents représentent un radical alcoyle renfermant de 1 à 8 atomes de carbone, ou avec un malonate d'alcoyle de formule

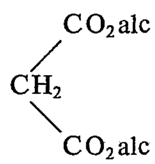

dans laquelle alc représente un radical alcoyle renfermant de 1 à 8 atomes de carbone, en présence d'une base forte, pour obtenir un composé de formule VI

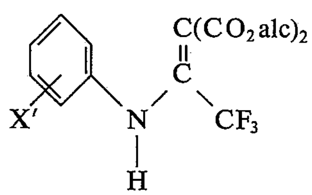

(VI)

que l'on cyclise pour obtenir un composé de formule VII

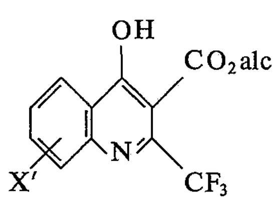

(VII)

que l'on saponifie pour obtenir un composé de formule II correspondant

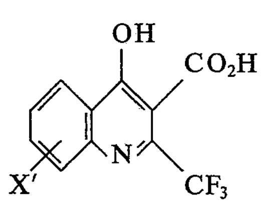

(II)

Dans un mode de réalisation préférée du procédé de préparation du composé de formule II:

- La réaction du composé de formule IV avec l'agent de chloruration qui est, de préférence, le pentachlorure de phosphore ou le mélange triphénylphosphine — tétrachlorure de carbone, a lieu à une température comprise entre 60 et 120° C.
- Le dérivé de formule

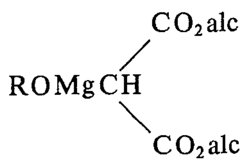

utilisé est un dérivé pour lequel R et alc représentent un radical méthyle, éthyle ou n-propyle.
- Le malonate d'alcoyle utilisé est le malonate d'éthyle, et la réaction avec le composé de formule V est effectuée en présence d'hydrure de sodium.
- La cyclisation du composé de formule VI est réalisée par chauffage, par exemple à une température comprise entre 150° C et 250° C.
- On saponifie le composé de formule VII au moyen de la soude ou de la potasse.

Les composés de formule IV utilisés comme produits de départ sont des produits connus d'une façon générale qui peuvent être préparés selon le procédé décrit dans CA 54 4430 i.

Les exemples suivants illustrent l'invention sans toutefois la limiter.

## Exemple 1

### 2,8-bis (trifluorométhyl) 4-hydroxy N-(2-thiazolyl) 3-quinoléinecarboxamide

On met en suspension 9,1 g de chlorure de l'acide 2,8 bis (trifluorométhyl) 4-hydroxy 3-quinoléine carboxylique obtenu selon le procédé décrit dans la demande divisionnaire de la présente demande (préparation I) publiée sous le n° 00 49555) dans 50 cm3 de pyridine. On ajoute à la solution précédente 2,66 g de 2-aminothiazole en solution dans 15 cm3 de pyridine. On maintient à la température ambiante pendant une nuit et évapore la pyridine sous pression réduite. On obtient ainsi 15,9 g d'un produit que l'on met en solution dans l'acétate d'éthyle. On extrait l'acide de départ au bicarbonate de soude, évapore l'acétate d'éthyle et purifie le produit obtenu par empâtage au chlorure de méthylène et par recristallisation dans l'acide acétique. On obtient ainsi 2,57 g du produit recherché fondant à 258° C.

## Exemple 2

### 2,8-bis (trifluorométhyl) 4-hydroxy N-(oxazol-2-yl) 3-quinoléine carboxamide

Sous agitation et courant d'azote à 5~8° C on ajoute 15,9 g du chlorure de l'acide 2,8-bis(trifluorométhyl) 4-hydroxy 3-quinoléine carboxylique en solution dans 210 cm3 d'acétate d'éthyle, une solution renfermant 3,57 g de 2-aminooxazol et 4,3 g de triéthylamine dans 50 cm3 d'acétate d'éthyle. On laisse revenir à la température ambiante et laisse reposer le mélange réactionnel pendant une nuit. On essore, filtre, lave le filtrat et évapore à sec sous pression réduite. On obtient ainsi 14,23 g du produit recherché brut que l'on triture dans 100 cm3 d'une solution normale de soude pendant trois heures puis dilue avec 200 cm3 d'eau. On essore l'insoluble, passe le filtrat au charbon actif, amène à pH 1 par addition à une solution normale d'acide chlorhydrique. On essore le précipité formé, le lave à l'eau, le dissout dans l'acétate d'éthyle et le sèche. On obtient 8,77 g d'un produit que l'on recristallise 2 fois dans l'acide acétique. On obtient ainsi 5,73 g de produit recherché fondant à 258° C (rendement total 30,8%).

## Exemple 3

### 2,8-bis (trifluorométhyl) 4-hydroxy N-(5-méthylisoxazol 3-yl) 3-quinoléine carboxamide

En opérant comme à l'exemple précédant, au départ de 27,2 g de chlorure de l'acide 2,8-bis (trifluorométhyl) 4-hydroxy 3-quinoléine carboxylique, et du 6,38 g de 3-amino 5-méthyl isoxazole on obtient 12,8 g du produit recherché fondant à 216° C.

## Exemple 4

### 2,8-bis (trifluorométhyl) 4-hydroxy N-(2-pyridinyl) 3-quinoléinecarboxamide

On ajoute à 8°C sous agitation et sous atmosphère d'azote a 13,4 g de chlorure de l'acide 2,8-bis (trifluorométhyl) 4-hydroxy 3-quinoléine carboxylique en suspension dans 225 cm3 de benzène, 3,29 g de 2-aminopyridine et 3,54 g de triéthylamine en solution dans 70 cm3 de benzène. On laisse revenir à la température ambiante et porte au reflux pendant une heure. On refroidit, ajoute 300 cm3 d'eau et 750 cm3 d'acétate d'éthyle. On filtre l'insoluble, et décante le filtrat. On extrait les phases organiques avec une solution de carbonate acide de sodium et les lave. On sèche, filtre et évapore à sec. On obtient 11,52 g d'un produit que l'on recristallise dans l'acide acétique. On obtient ainsi 5,77 g du produit recherché fondant à 236° C.

## Exemple 5

### 2,8-bis (trifluorométhyl) 4-hydroxy N-(1-méthyl-IH imidazol-2-yl) 3-quinoléine carboxamide

En opérant comme à l'exemple 1, à partir du chlorure de l'acide 2,8-bis (trifluorométhyl) 4-hydroxy 3-quinoléine carboxylique et du 2-amino N-méthyl imidazole. On obtient le produit recherché fondant à 259° C avec un rendement de 33,9%.

## Exemple 6

### 2,8-bis-(trifluorométhyl) 4-hydroxy N-(3-chlorophényl) 3-quinoléine carboxamide

On dissout 4,083 g de métachloro aniline dans 9,71 g de triéthylamine et 65 cm3 d'acétate d'éthyle, verse, en 1/4 heure, dans une solution de 11,96 g de chlorure d'acide 2,8-bis-(trifluorométhyl) 4-hydroxy 3-quinoléine carboxylique dans 130 cm3 d'acétate d'éthyle, maintenue à 8—10°C et agitée vivement. On laisse revenir à température ambiante et porte au reflux pendant 1 heure. Après refroidissement, on essore l'insoluble et lave le filtrat à l'eau, sèche, évapore à sec à 40°C sous pression réduite et obtient 17,14 g de sel de triéthylamine du produit recherché. On reprend par la soude 2N, porte à ébullition et après retour à température ambiante, on essore le sel de sodium du produit recherché que l'on dissout dans l'eau, acidifie à pH 1 avec de l'acide chlorhydrique à 20% et essore le précipité. Après recristallisation dans le benzène, on obtient 7,599 g de produit attendu. F. = 152° C.

## Exemple 7

### 2,8-bis-(trifluorométhyl) 4-hydroxy N-(1H-tétrazol 5-yl) 3-quinoléine carboxamide

On refroidit à 8°C, tout en agitant, 5,03 g de 5-amino tétrazole anhydre dans 230 cm3 d'acétate d'éthyle. Dans cette suspension, on introduit simultanément, en 1/4 heure, une solution de 21,9 g de chlorure d'acide 2,8-bis-(trifluorométhyl) 4-hydroxy 3-quinoléine carboxylique dans 230 cm3 d'acétate d'éthyle et une solution de 18 g de triéthylamine dans 180 cm3 d'acetate d'éthyle. On laisse revenir à température ambiante, puis chauffe au reflux pendant une heure. Après refroidissement, on essore l'insoluble, le dissout dans 1 litre d'eau, ajoute les eaux de lavage du filtrat, amène à pH 1 avec de l'acide chlorhydrique à 20% et essore le précipité formé. On le dissout dans 50 cm3 de soude 2N, dilue avec 150 cm3 d'eau et porte à ébullition. On refroidit, acidifie avec 100 cm3 d'acide chlorhydrique à 20%, essore, sèche et recristallise dans 250 cm3 d'acide acétique. On obtient 12,279 g de produit attendu. F. = > 260° C.

Analyse: $C_{13}H_6F_6N_6O_2$ PM: 392,22

| | | | | |
|---|---|---|---|---|
| Calculé: | C 39,81 | H 1,54 | N 21,43 | F 29,06% |
| Trouvé: | C 39,6 | H 1,6 | N 22,0 | F 28,1% |

## Exemple 8

### 2,7-bis-(trifluorométhyl) 4-hydroxy N-(2-thiazolyl) 3-quinoléine carboxamide

A une solution de 18,62 g de chlorure d'acide 2,7-bis-(trifluorométhyl) 4-hydroxy 3-quinoléine carboxylique obtenu selon le procédé décrit dans la demande divisionnaire de la présente demande

(préparation II), publiée sous le n° 00 49555) dans 350 cm3 d'acétate d'éthyle, maintenue à 7°C, on ajoute, sous agitation, en 25 minutes, une solution constituée par 5,37 g de 2-aminothiazole, 16,27 g de triéthylamine et 214 cm3 d'acétate d'éthyle. On laisse revenir à température ambiante et porte au reflux pendant une heure. Après refroidissement, on essore l'insoluble, lave à l'eau le filtrat, sèche, évapore à sec à 40°C sous pression réduite et obtient 19,22 g de produit recherché sous forme de sel de triéthylamine. On reprend celui-ci par 180 cm3 de soude 2N, chauffe à ébullition pendant 3 ou 4 minutes, refroidit et essore l'insoluble. Le filtrat est amené à pH 1 avec de l'acide chlorhydrique à 20%. On essore le précipité formé et recristallise dans l'acide acétique. On obtient 8,22 g de produit attendu. F. = 270°C.

## Exemple 9

### 2-(trifluorométhyl) 4-hydroxy N-(2-thiazolyl) 3-quinoléine carboxamide

On dissout le chlorure d'acide 2-(trifluorométhyl) 4-hydroxy 3-quinoléine carboxamide obtenu selon le procédé décrit dans la demande divisionnaire de la présente demande (préparation III) publiée sous le n° 00 49555), dans 120 cm3 de pyridine anhydre, ajoute 7,5 g de 2-aminothaizole et 20 cm3 de pyridine. On chauffe à 80—85°C pendant 1 heure et agite 16 heures à température ambiante. Après concentration à sec du mélange réactionnel, on triture le résidu dans 200 cm3 d'eau, essore l'insoluble, le lave réunit les eaux de lavage et le filtrat, refroidit dans la glace, acidifie avec de l'acide chlorhydrique concentré, essore le précipité, lave à l'eau, sèche et obtient 13,7 g de produit attendu. F. > 280°C.

## Exemple 10

### 2-trifluorométhyl 6-isopropyl 4-hydroxy N-(2-thiazolyl) 3-quinoléine carboxamide

On opère au départ du chlorure d'acide 2-trifluorométhyl 6-isopropyl 4-hydroxy 3-quinoléine carboxylique obtenu selon le procédé décrit dans la demande divisionnaire de la présente demande (préparation IV) publiée sous le n° 0049555) et de 10 g de 2-aminothiazole, de la manière décrite à l'exemple 1. On obtient après recristallisation dans l'isopropanol 9,64 g de produit attendu. F. = 262°C.

## Exemple 11

### 2-trifluorométhyl 6-méthoxy 4-hydroxy N-(2-thiazolyl) 3-quinoléine carboxamide

On opère au départ du chlorure d'acide 2-trifluorométhyl 6-méthoxy 4-hydroxy 3-quinoléine carboxylique obtenu selon le procédé décrit dans la demande divisionnaire de la présente demande (préparation V) publiée sous le n° 0049555), et de 1,05 g de 2-aminothiazole, de la manière décrite à l'exemple 1. On obtient après recristallisation dans l'éthanol 1,6 g de produit attendu. F. > 270°C.

## Exemple 12

### Compositions pharmaceutiques

On a préparé des comprimés répondant à la formule suivante:

| | |
|---|---|
| Produit de l'exemple 1 | 50 mg |
| Excipient q.s. pour un comprimé terminé à | 350 mg |

(Détail de l'excipient: lactose, talc, amidon, stéarate de magnésium).

## Etude pharmacologique du produit de l'exemple 1

### 1. Etude de l'activité analgésique

Le test employé est basé sur le fait signalé par R. KOSTER et Coll., (Fed, Proc., 1959, 1B, 412) selon lequel l'injection intrapéritonéale d'acide acétique provoque, chez la souris, des mouvements répétés d'étirement et de torsion pouvant persister pendant plus de six heures. Les analgésiques préviennent ou diminuent ce syndrome qui peut être considéré comme l'extériorisation d'une douleur abdominale diffuse. On utilise une solution d'acide acétique à 1% dans l'eau. La dose déclenchant le syndrome est

dans ces conditions de 0,01 cm3/g, soit 100 mg/kg d'acide acétique.

Le produit étudié est administré par voie buccale une demiheure avant l'injection d'acide acétique, les souris étant à jeun depuis la veille de l'expérience.

Les étirements sont observés et comptés pour chaque souris, pendant une période d'observation de quinze minutes commençant aussitôt après l'injection d'acide acétique.

Les résultats sont exprimés au moyen de la $DA_{50}$, c'est-à-dire la dose qui permet d'obtenir une diminution de 50% du nombre des étirements par rapport aux animaux témoins.

La $DA_{50}$ trouvée a été de 0,55 mg/kg.

2. Etude de l'activité anti-inflammatoire

L'activité anti-inflammatoire a été déterminée sur le test de l'arthrite provoquée par la carraghénine chez le rat.

On administre, à des rats mâles pesant de 130 à 150 g, 0,05 cm3 d'une suspension stérile à 1% de carraghénine dans l'articulation tibio-tarsienne d'une patte postérieure.

Simultanément, on administre le produit à étudier dans une suspension de carboxyméthylcellulose à 0,25% et de Tween à 0,02% par voie orale.

Le volume de la patte est mesuré avant l'administration, puis deux heures, quatre heures, six heures, huit heures et vingt-quatre heures après.

L'intensité de l'inflammation est maxima quatre à six heures après l'injection de carraghénine. La différence du volume des pattes des animaux traités et des témoins met en évidence l'action anti-inflammatoire du médicament.

On détermine la $DA_{50}$, c'est-à-dire la dose qui permet d'obtenir une diminution de l'oedème de 50%.

Pour le produit de l'exemple 1, la $DA_{50}$ a été trouvée de 4 mg/kg.

**Revendications**

1. Les composés de formule I':

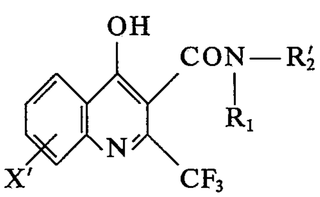

(I')

dans laquelle X' en position 5, 6, 7 ou 8 représente un atome d'hydrogène, un atome d'halogène, un radical alcoyle linéaire ou ramifié renfermant de 1 à 5 atomes de carbone, un radical alcoxy linéaire ou ramifié renfermant de 1 à 4 atomes de carbone, un radical trifluorométhyle, un radical trifluorométhylthio ou un radical trifluorométhoxy, $R_1$ représente un atome d'hydrogène ou un radical alcoyle renfermant de 1 à 4 atomes de carbone, $R'_2$ représente un radical choisi parmi les radicaux thiazolyle, 4,5-dihydrothiazolyle, pyridinyle, oxazolyle, isoxazolyle, imidazolyle, pyrimidyle ou tétrazolyle, éventuellement substitués par un radical alcoyle renfermant de 1 à 4 atomes de carbone et phényle, éventuellement substitué, par au moins un radical choisi dans le groupe formé par les radicaux alcoyles renfermant de 1 à 4 atomes de carbone, les radicaux alcoxy renfermant de 1 à 4 atomes de carbone, le radical trifluorométhyle, le radical nitro et les atomes d'halogène, ainsi que leurs sels d'addition avec les acides.

2. Les composés selon la revendication 1, répondant à la formule I:

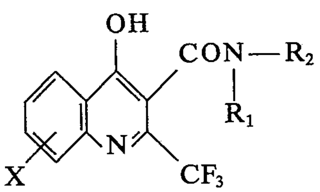

(I)

dans laquelle X en position 5, 6, 7 ou 8 représente un atome d'halogène, un radical trifluorométhyle, un radical trifluorométhylthio ou un radical trifluorométhoxy, $R_1$ représente un atome d'hydrogène ou un radical alcoyle renfermant de 1 à 4 atomes de carbone, $R_2$ représente un radical hétérocyclique choisi parmi les radicaux thiazolyle, 4,5-dihydrothiazolyle, pyridinyle, oxazolyle, isoxazolyle, imidazolyle ou pyrimidyle, éventuellement substitués par un radical alcoyle renfermant de 1 à 4 atomes de carbone, ainsi que leurs sels d'addition avec les acides.

3. Les composés de formule I', tels que définis à la revendication 1 ou 2, pour lesquels X' représente

un radical trifluorométhyle, ainsi que leurs sels d'addition avec les acides.

4. Les composés de formule I', tels que définis à la revendication 1, 2 ou 3, pour lesquels X' est en position 8, ainsi que leurs sels d'addition avec les acides.

5. Les composés de formule I', tels que définis à l'une quelconque des revendications 1 à 4, pour lesquels $R_1$ représente un atome d'hydrogène, ainsi que leurs sels d'addition avec les acides.

6. Les composés de formule I', tels que définis à l'une quelconque des revendications 1 à 5, pour lesquels $R'_2$ représente un radical thiazolyle, ainsi que leurs sels d'addition avec les acides.

7. Les composés de formule I', tels que définis à l'une quelconque des revendications 1 à 5, pour lesquels $R'_2$ représente un radical oxazolyle, ainsi que leurs sels d'addition avec les acides.

8. Les composés de formule I', tels que définis à l'une quelconque des revendications 1 à 5, pour lesquels $R'_2$ représente un radical isocazolyle éventuellement substitué par un radical alcoyle renfermant de 1 à 4 atomes de carbone, ainsi que leurs sels d'addition avec les acides.

9. Les composés de formule I', tels que définis à l'une quelconque des revendications 1 à 5, pour lesquels $R'_2$ représente le radical 5-méthyl isoxazolyle, ainsi que leurs sels d'addition avec les acides.

10. Les composés de formule I', tels que définis à l'une quelconque des revendications 1 à 5, pour lesquels $R'_2$ représente un radical pyrimidyle, ainsi que leurs sels d'addition avec les acides.

11. Les composés de formule I' tels que définis à l'une quelconque des revendications 1 à 5, pour lesquels $R'_2$ représente un radical imidazolyle éventuellement substitué par un radical alcoyle renfermant de 1 à 4 atomes de carbone.

12. Les composés de formule I', tels que définis à l'une quelconque des revendications 1 à 5, pour lesquels $R'_2$ représente le radical 1-méthyl 1H-imidazolyle ainsi que leurs sels d'addition avec les acides.

13. Les composés de formule I', tels que définis à l'une quelconque des revendications 1 et 3 à 5, pour lesquels $R'_2$ représente un radical tétrazolyle éventuellement substitué par un radical alcoyle renfermant de 1 à 4 atomes de carbone.

14. A titre de médicament, des composés de formule I', tels que définis à l'une quelconque des revendications 1 à 13, ainsi que leurs sels d'addition avec les acides minéraux ou organiques thérapeutiquement compatibles.

15. A titre de médicament, le 2,8 bis-(trifluorométhyl) 4-hydroxy N-(2-thiazolyl) 3-quinoléine carboxamide.

16. Les compositions pharmaceutiques renfermant comme principe actif au moins un médicament défini à la revendication 14 ou 15.

17. Procédé de préparation des composés de formule I' définis à l'une quelconque des revendications 1 à 13 ainsi que leurs sels d'addition avec les acides, caractérisé en ce que l'on soumet un acide de formule II ou un dérivé fonctionnel de ce dernier

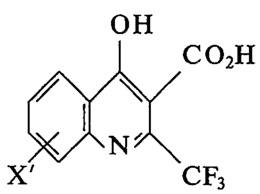

(II)

dans laquelle X' conserve la même signification que dans la revendication 1, à l'action d'un dérivé de formule III:

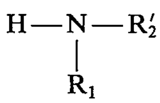

(III)

dans laquelle $R_1$ et $R'_2$ sont définis comme dans la revendication 1, pour obtenir le composé de formule I' correspondant, que l'on soumet, si désiré, à l'action d'un acide pour en former le sel.

## Patentansprüche

1. Verbindungen der Formel I'

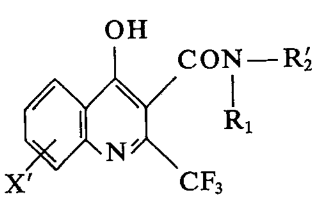

(I')

worin X′ in 5-, 6-, 7- oder 8-Stellung ein Wasserstoffatom, ein Halogenatom, einen linearen oder verzweigten Alkylrest mit 1 bis 5 Kohlenstoffatomen, einen linearen oder verzweigten Alkoxyrest mit 1 bis 4 Kohlenstoffatomen, einen Trifluormethylrest, einen Trifluormethylthiorest oder einen Trifluormethoxyrest bedeutet, $R_1$ ein Wasserstoffatom oder einen Alkylrest mit 1 bis 4 Kohlenstoffatomen bedeutet, $R'_2$ einen Rest, ausgewählt unter den Thiazolyl-, 4,5-Dihydrothiazolyl-, Pyridinyl-, Oxazolyl-, Isoxazolyl-, Imidazolyl-, Pyrimidyl- oder Tetrazolylresten, die gegebenenfalls substituiert sind durch einen Alkylrest mit 1 bis 4 Kohlenstoffatomen, und Phenyl, gegebenenfalls substituiert durch zumindest einen Rest, ausgewählt unter den Alkylresten mit 1 bis 4 Kohlenstoffatomen, den Alkoxyresten mit 1 bis 4 Kohlenstoffatomen, dem Trifluormethylrest, dem Nitrorest und den Halogenatomen, bedeutet sowie deren Additionssalze mit Säuren.

2. Verbindungen gemäß Anspruch 1 der Formel I

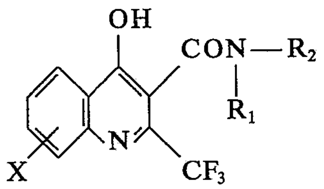

(I)

worin X in 5-, 6-, 7- oder 8-Stellung ein Halogenatom, einen Trifluormethylrest, einen Trifluormethylthiorest oder einen Trifluormethoxyrest bedeutet, $R_1$ ein Wasserstoffatom oder einen Alkylrest mit 1 bis 4 Kohlenstoffatomen bedeutet, $R_2$ einen heterocyclischen Rest, ausgewählt unter den Thiazolyl-, 4,5-Dihydrothiazolyl-, Pyridinyl-, Oxazolyl-, Isoxazolyl-, Imidazolyl- oder Pyrimidylresten, die gegebenenfalls durch einen Alkylrest mit 1 bis 4 Kohlenstoffatomen substituiert sind, bedeutet sowie deren Additionssalze mit Säuren.

3. Verbindungen der Formel I′ gemäß Anspruch 1 oder 2, worin X′ einen Trifluormethylrest bedeutet, sowie deren Additionssalze mit Säuren.

4. Verbindungen der Formel I′ gemäß Anspruch 1, 2 oder 3, worin sich X′ in 8-Stellung befindet, sowie deren Additionssalze mit Säuren.

5. Verbindungen der Formel I′ gemäß einem der Ansprüche 1 bis 4, worin $R_1$ ein Wasserstoffatom bedeutet, sowie deren Additionssalze mit Säuren.

6. Verbindungen der Formel I′ gemäß einem der Ansprüche 1 bis 5, worin $R'_2$ einen Thiazolylrest bedeutet, sowie deren Additionssalze mit Säuren.

7. Verbindungen der Formel I′ gemäß einem der Ansprüche 1 bis 5, worin $R'_2$ einen Oxazolylrest bedeutet, sowie deren Additionssalze mit Säuren.

8. Verbindungen der Formel I′ gemäß einem der Ansprüche 1 bis 5, worin $R'_2$ einen Isoxazolylrest bedeutet, der gegebenenfalls durch einen Alkylrest mit 1 bis 4 Kohlenstoffatomen substituiert ist, sowie deren Additionssalze mit Säuren.

9. Verbindungen der Formel I′ gemäß einem der Ansprüche 1 bis 5, worin $R'_2$ den 5-Methylisoxazolylrest bedeutet, sowie deren Additionssalze mit Säuren.

10. Verbindungen der Formel I′ gemäß einem der Ansprüche 1 bis 5, worin $R'_2$ einen Pyrimidylrest bedeutet, sowie deren Additionssalze mit Säuren.

11. Verbindungen der Formel I′ gemäß einem der Ansprüche 1 bis 5, worin $R'_2$ einen Imidazolylrest bedeutet, der gegebenenfalls durch einen Alkylrest mit 1 bis 4 Kohlenstoffatomen substituiert ist.

12. Verbindungen der Formel I′ gemäß einem der Ansprüche 1 bis 5, worin $R'_2$ den 1-Methyl-1H-imidazolylrest bedeutet, sowie deren Additionssalze mit Säuren.

13. Verbindungen der Formel I′ gemäß einem der Ansprüche 1 und 3 bis 5, worin $R'_2$ einen Tetrazolylrest bedeutet, der gegebenenfalls durch einen Alkylrest mit 1 bis 4 Kohlenstoffatomen substituiert ist.

14. Als Arzneimittel die Verbindungen der Formel I′ gemäß einem der Ansprüche 1 bis 13, sowie deren Additionssalze mit therapeutisch verträglichen Mineral- oder organischen Säuren.

15. Als Arzneimittel das 2,8-Bis-(trifluormethyl)-4-hydroxy-N-(2-thiazolyl)-3-chinolincarboxamid.

16. Pharmazeutische Zusammensetzungen, enthaltend als Wirkstoff zumindest ein Arzneimittel gemäß Anspruch 14 oder 15.

17. Verfahren zur Herstellung der Verbindungen der Formel I′ gemäß einem der Ansprüche 1 bis 13, sowie von deren Additionssalzen mit Säuren, dadurch gekennzeichnet, daß man eine Säure der Formel II oder ein funktionelles Derivat dieser letzteren

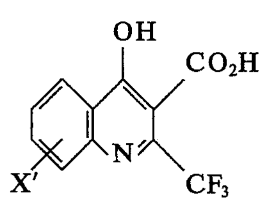

(II)

worin X' die in Anspruch 1 angegebene **Bedeutung besitzt, der Einwirkung eines Derivats der Formel III**

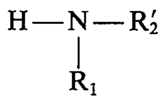

(III)

unterzieht, worin $R_1$ und $R'_2$ wie in Anspruch 1 definiert sind, um die entsprechende Verbindung der Formel I' zu erhalten, die man gewünschtenfalls der Einwirkung einer Säure unterzieht, um hieraus das Salz zu bilden.

## Claims

1. The compounds with the formula I':

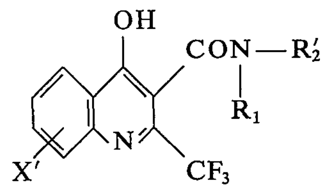

(I')

in which X' in position 5, 6, 7 or 8 represents a hydrogen atom, a halogen atom, a linear or branched alkyl radical containing 1 to 5 carbon atoms, a linear or branched alkoxy radical containing from 1 to 4 carbon atoms, a trifluoromethyl radical, a trifluoromethylthio radical or a trifluoromethoxy radical, $R_1$ represents a hydrogen atom or an alkyl radical containing from 1 to 4 carbon atoms, $R'_2$ represents a radical chosen from thiazolyl, 4,5-dihydrothiazolyl, pyridinyl, oxazolyl, isoxazolyl, imidazolyl, pyrimidyl or tetrazolyl radicals, possibly substituted by an alkyl radical containing from 1 to 4 carbon atoms, and phenyl, possibly substituted by at least one radical chosen from the group formed by the alkyl radicals containing from 1 to 4 carbon atoms, the alkoxy radicals containing from 1 to 4 carbon atoms, a trifluoromethyl radical, a nitro radical and halogen atoms, as well as their salts of addition with acids.

2. The compounds according to Claim 1, corresponding to the formula I:

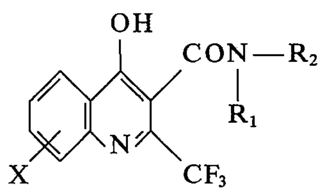

(I)

in which X in position 5, 6, 7 or 8 represents a halogen atom, a trifluoromethyl radical, a trifluoromethylthio radical, or a trifluoromethoxy radical, $R_1$ represents a hydrogen atom or an alkyl radical containing from 1 to 4 carbon atoms, $R_2$ represents a heterocyclic radical chosen from thiazolyl, 4,5-dihydrothiazolyl, pyridinyl, oxazolyl, isoxazolyl, imidazolyl or pyrimidyl radicals, possibly substituted by an alkyl radical containing from 1 to 4 carbon atoms, as well as their salts of addition with acids.

3. The compounds with the formula I', as defined in Claim 1 or 2, for which X' represents a trifluoromethyl radical, as well as their salts of addition with acids.

4. The compounds with the formula I', as defined in Claim 1, 2 or 3, for which X' is in position 8, as well as their salts of adddition with acids.

5. The compounds with the formula I', as defined in any one of the Claims 1 to 4, for which $R_1$ represents a hydrogen atom, as well as their salts of addition with acids.

6. The compounds with the formula I', as defined in any one of the Claims 1 to 5, for which $R'_2$ represents a thiazolyl radical, as well as their salts of addition with acids.

7. The compounds with the formula I', as defined in any one of the Claims 1 to 5, for which $R'_2$ represents an oxazolyl radical, as well as their salts of addition with acids.

8. The compounds with the formula I', as defined in any one of the Claims 1 to 5, for which $R'_2$ represents an isoxazolyl radical possibly substituted by an alkyl radical containing from 1 to 4 carbon atoms, as well as their salts of addition with acids.

9. The compounds with the formula I', as defined in any one of the Claims 1 to 5, for which $R'_2$ represents the 5-methyl isoxazolyl as well as their salts of addition with acids.

10. The compounds with the formula I', as defined in any one of the Claims 1 to 5, for which $R'_2$ represents a pyrimidyl radical, as well as their salts of addition with acids.

11. The compounds with the formula I' as defined in any one of the Claims 1 to 5, for which $R'_2$

represents an imidazolyl radical, possibly substituted by an alkyl radical containing from 1 to 4 carbon atoms.

12. The compounds with the formula I', as defined in any one of the Claims 1 to 5, for which $R'_2$ represents the 1-methyl 1H-imidazolyl radical as well as their salts of addition with acids.

13. The compounds with the formula I', as defined in any one of the Claims 1 and 3 to 5, for which $R'_2$ represents a tetrazolyl radical possibly substituted by an alkyl radical containing from 1 to 4 carbon atoms.

14. As a medicament, the compounds with the formula I', as defined in any one of the Claims 1 to 13, as well as their salts of addition with therapeutically compatible mineral or organic acids.

15. As medicament, 2,8-bis-(trifluoromethyl) 4-hydroxy N-(2-thiazolyl) 3-quinolein carboxamide.

16. The pharmaceutical compositions containing as active principle at least one medicament defined in Claim 14 or 15.

17. Process for the preparation of the compounds with the formula I' defined in any one fo the Claims 1 to 13, as well as their salts of addition with acids, characterized in that an acid with the formula II or a functional derivative thereof

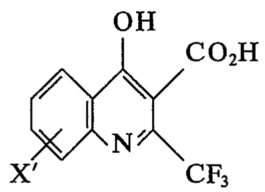

(II)

in which X' retains the same significance as in Claim 1, is submitted to the action of a derivative with the formula III:

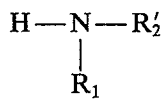

in which $R_1$ and $R'_2$ are defined as in Claim 1, as to obtain the corresponding compound with the formula I', which, if desired, is submitted to the action of an acid in order to form the salt.